# DEMANDE DE BREVET EUROPEEN

(11) **EP 2 821 476 A1**
(43) Date de publication de la demande: **07.01.2015**
(21) Numéro de dépôt: 14175882.1
(22) Date de dépôt: 04.07.2014
(51) Int. Cl.: C12M 1/107, C12M 1/00

(54) **Digesteur pour la production de biogaz et de digestat et son procédé de montage**

(30) Priorité: 04.07.2013 FR 1356585
(71) Demandeur: Valogreen, 60240 Reilly (FR)
(72) Inventeur: Delacour, Thomas, 60240 Reilly (FR)
(74) Mandataire: Cabinet Chaillot

(57) **Abrégé**

L'invention a pour objet un digesteur (1) pour la production de biogaz (6) et de digestat, comprenant un silo externe (2) dans lequel est logé un réceptacle pour les matières organiques fermentescibles (5), dont la fermentation conduit d'une part à un digestat restant dans ledit réceptacle et, d'autre part à un biogaz (6) se formant au-dessus desdites matières organiques fermentescibles qui se transforment en digestat, ledit silo (2) comportant au moins une entrée desdites matières organiques fermentescibles et au moins une sortie dudit biogaz (6), caractérisé par le fait que le réceptacle pour les matières organiques fermentescibles consiste en une enveloppe souple (4), fermée sur elle-même, de confinement desdites matières organiques fermentescibles (5) et dudit biogaz (6), la ou les entrées de matières organiques fermentescibles (5) et la ou les sorties de biogaz (6) traversant ladite enveloppe (4) de manière étanche.

## Description

La présente invention concerne la valorisation de matières organiques fermentescibles telles que les lisiers, les fumiers, les tontes de pelouses, les résidus et excédents de récoltes comme les menues pailles, l'ensilage et leurs mélanges.

L'invention a pour objet un digesteur (aussi appelé méthaniseur) dans lequel ces matières organiques fermentescibles sont introduites et se décomposent par méthanisation, donnant, d'une part, un biogaz qui est un mélange gazeux formé essentiellement de méthane et de dioxyde de carbone, et, d'autre part, un digestat qui est utile comme biofertilisant. Un exemple de composition de biogaz est CH₄+CO₂+impuretés : 59/40/1 en volume.

L'invention a également pour objet un procédé de montage de ce digesteur.

Le déploiement de la méthanisation dans les exploitations et fermes agricoles est un enjeu économique très important. En effet, la méthanisation agricole permet :
- de traiter simultanément toutes les matières organiques fermentescibles, telles que mentionnées ci-dessus, qui sont des déchets et des surplus de récoltes ;
- de générer un flux continu de fertilisant pour ces exploitations et fermes agricoles, remplaçant les fertilisants minéraux traditionnels qui proviennent du pétrole et du gaz naturel et produisent des gaz à effet de serre dans une bien plus grande mesure ;
- de fournir des ressources énergétiques d'appoint : gaz méthane, carburants, électricité, compléments de chaleur par le brûlage de CH₄ en CO₂ pour les bâtiments de la ferme, les logements proches et activités nécessitant de la chaleur telles que le chauffage de serres, de piscines, le séchage, etc.

Les digesteurs connus consistent en des silos dans lesquels sont logés des réceptacles pour les matières organiques fermentescibles, la fermentation conduisant au dégagement de biogaz au-dessus des matières organiques en fermentation.

Ces installations sont cependant des installations importantes nécessitant beaucoup de génie civil et des dispositifs complexes pour la récupération du biogaz dans les conditions de sécurité requises. De plus, leur efficacité biologique n'est pas optimale.

La Société déposante a cherché à remédier à ces inconvénients et propose, selon la présente invention, une nouvelle architecture de digesteur constituée par un silo classique renfermant un réceptacle à la fois pour les matières organiques fermentescibles et le biogaz se formant au-dessus de celles-ci, un tel réceptacle étant formé d'une enveloppe souple.

Une telle architecture présente l'avantage d'un montage simple, rapide, performant et durable, avec un silo robuste et économique, de type de ceux utilisés de façon classique dans le monde agricole, facilement isolable par une isolation assurée notamment par de la mousse de polyuréthane le long de sa paroi interne et sur son fond, et avec le digesteur proprement dit et son gazomètre réalisés par une enveloppe souple assurant à la fois le réceptacle des matière en fermentation et la couverture du ciel gazeux, et venant en appui contre le fond et la paroi interne dudit silo.

Un gain de place est également assuré par rapport aux digesteurs traditionnels qui, comme déjà indiqué, sont des ouvrages imposants.

De plus, l'efficacité biologique peut être optimale du fait que l'espace de stockage de biogaz est délimité par l'enveloppe-même qui sert de rétention pour les matières en fermentation et le digestat, l'enveloppe étant libre et souple dans sa partie supérieure pour servir de gazomètre et pouvant être fixée au toit du silo pour ne pas venir toucher le digestat. L'enveloppe peut avantageusement être choisie en un matériau tel une géomembrane résistante dans le temps et résistant aux agressions chimiques et physiques, telles que la température, une tension, un déchirement, un poinçonnement, une élasticité.

L'étanchéité de la structure selon l'invention à la fois aux liquides et aux gaz s'effectue par l'intermédiaire de l'enveloppe souple placée à l'intérieur du silo.

Un chauffage de l'ensemble peut être effectué par une circulation d'eau chaude, issue avantageusement d'un moteur de cogénération, dans un réseau de serpentins situé contre l'enveloppe souple entre l'isolation et ladite enveloppe souple, un système de diffusion de chaleur formé d'une feuille métallique permettant un meilleur transfert thermique.

Ainsi, le dispositif de l'invention permet de répondre aux besoins de simplification, de réduction d'encombrement, de rendement et de sécurité qui se posaient pour les digesteurs connus.

La présente invention a donc d'abord pour objet un digesteur pour la production de biogaz et de digestat, comprenant un silo externe dans lequel est logé un réceptacle pour les matières organiques fermentescibles, dont la fermentation conduit d'une part à un digestat restant dans ledit réceptacle et, d'autre part à un biogaz se formant au-dessus desdites matières organiques fermentescibles qui se transforment en digestat, ledit silo comportant au moins une entrée desdites matières organiques fermentescibles et au moins une sortie dudit biogaz, caractérisé par le fait que le réceptacle pour les matières organiques fermentescibles consiste en une enveloppe souple, fermée sur elle-même, de confinement desdites matières organiques fermentescibles et dudit biogaz, la ou les entrées de matières organiques fermentescibles et la ou les sorties de biogaz traversant ladite enveloppe de manière étanche.

L'enveloppe souple est avantageusement une bâche souple ou une géomembrane consistant en une toile ou un tissu ou géotextile enduit notamment d'un polychlorure de vinyle(PVC).

En particulier, l'enveloppe souple peut être formée par l'assemblage de pièces par soudage.

Dans le cas où le digesteur comporte un silo externe de forme générale cylindrique, l'enveloppe souple peut être formée par soudage d'un fond circulaire, d'une bande rectangulaire soudée le long de ses deux bordures pour former une paroi latérale, et d'au moins une pièce de couverture.

La partie de couverture de l'enveloppe souple se situant au-dessus du biogaz peut porter au moins une sangle de liaison à un toit du silo externe.

L'enveloppe souple peut comporter dans sa partie de couverture au moins une ouverture refermable hermétiquement et le silo externe peut comporter dans un toit de celui-ci au moins un trou d'homme en regard d'une ouverture refermable hermétiquement.

L'enveloppe souple peut porter intérieurement, rattaché par points ou par lignes, à sa partie de couverture, un filet pour la fixation de bactéries qui se développent dans le biogaz et qui ont la capacité de faire précipiter le soufre présent dans celui-ci.

Une couche de matière isolante peut être formée entre le silo externe et l'enveloppe de confinement, excepté entre la partie de couverture de l'enveloppe et le toit du silo. Un dispositif de chauffage consistant en un serpentin hélicoïdal dans lequel circule de l'eau chaude peut être incorporé dans ladite couche de matière isolante de façon à entourer la paroi latérale de l'enveloppe, une rainure hélicoïdale étant pratiquée dans ladite couche de matière isolante pour recevoir le serpentin et un film mince métallique conducteur étant avantageusement appliqué au fond de ladite rainure.

La présente invention a également pour objet un procédé de montage d'un digesteur tel que défini ci-dessus, caractérisé par le fait que :
- on coule sur le sol une dalle de béton, on monte un silo sur la dalle de béton et on fait déboucher un tuyau dans le silo à travers la dalle de béton sur laquelle on a posé une plaque isolante, on pose une enveloppe souple non gonflée sur ladite plaque ;
- on raccorde ladite enveloppe et ledit tuyau pour que celui-ci débouche de manière étanche dans ladite enveloppe et on complète l'isolation du fond et de la paroi du silo, on pratique des trous dans l'isolant en regard des ouvertures prévues dans le silo pour l'entrée des matières fermentescibles et la sortie du biogaz ;
- on referme ces ouvertures et on place contre la paroi latérale de l'isolation un serpentin de passage d'eau chaude de préférence avec interposition d'une feuille métallique entre ladite paroi et le serpentin ;
- on gonfle l'enveloppe par un gonfleur à air placé sur le tuyau débouchant dans celle-ci ;
- on raccorde l'enveloppe de manière étanche sur les ouvertures précitées et on continue à gonfler ladite enveloppe ; et
- on attache ladite enveloppe au silo en partie supérieure.

Pour mieux illustrer l'objet de la présente invention, on va en décrire ci-après, à titre indicatif et non limitatif, plusieurs modes de réalisation particuliers avec référence au dessin annexé.

Sur ces dessins :
- la Figure 1 est une vue schématique en coupe axiale longitudinale pour illustrer le principe sur lequel est construit le digesteur selon la présente invention ;
- la Figure 2 est une vue analogue à la Figure 1 pour représenter un mode de réalisation de digesteur comportant un dispositif de chauffage ;
- la Figure 2a est, à plus grande échelle, une vue d'un détail de la Figure 2 ;
- la Figure 3 est une vue schématique en coupe partielle de la partie supérieure d'un digesteur conforme à un mode de réalisation particulier de la présente invention ;
- la Figure 4 est une vue schématique en perspective d'une enveloppe de confinement des déchets organiques fermentescibles et du biogaz dans sa position déployée ;
- les Figures 5 à 10 sont des vues illustrant les étapes successives du montage du digesteur conforme au mode de réalisation précité.
- La Figure 11 est une vue présentant le fonctionnement du digesteur en exploitation.

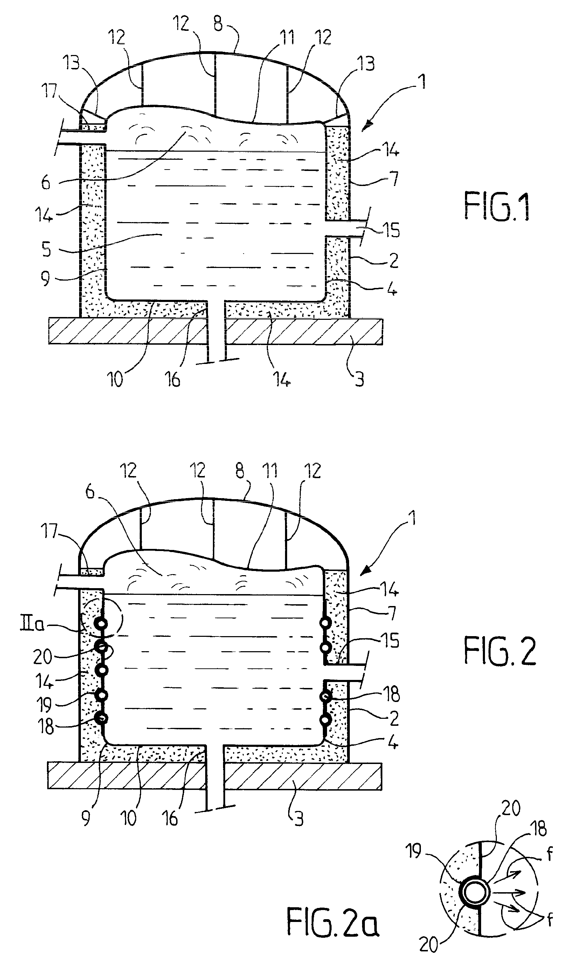

Si l'on se réfère à la Figure 1, on peut voir que l'on a désigné par 1 dans son ensemble un digesteur comportant un silo 2 qui est érigé sur une dalle 3 et dans lequel est logée une enveloppe souple 4 de confinement de déchets organiques fermentescibles 5 surmontés par un ciel gazeux 6.

Le silo 2 est réalisé en un matériau métallique, rigide, résistant aux forces de pression qui se développeront dans l'enveloppe 4. Ce matériau est typiquement un acier, par exemple un acier galvanisé, tel qu'utilisé de façon générale dans les installations de type silo.

La dalle 3 est une dalle de béton, en particulier une dalle coulée de béton armé.

Le silo 2 comporte une paroi latérale 7, avantageusement cylindrique, dont la base est fixée par des chevilles/étrier dans la dalle 3 et qui est fermée à sa partie supérieure par un toit 8 dont la forme est librement choisie par l'utilisateur, n'étant pas caractéristique du digesteur selon l'invention.

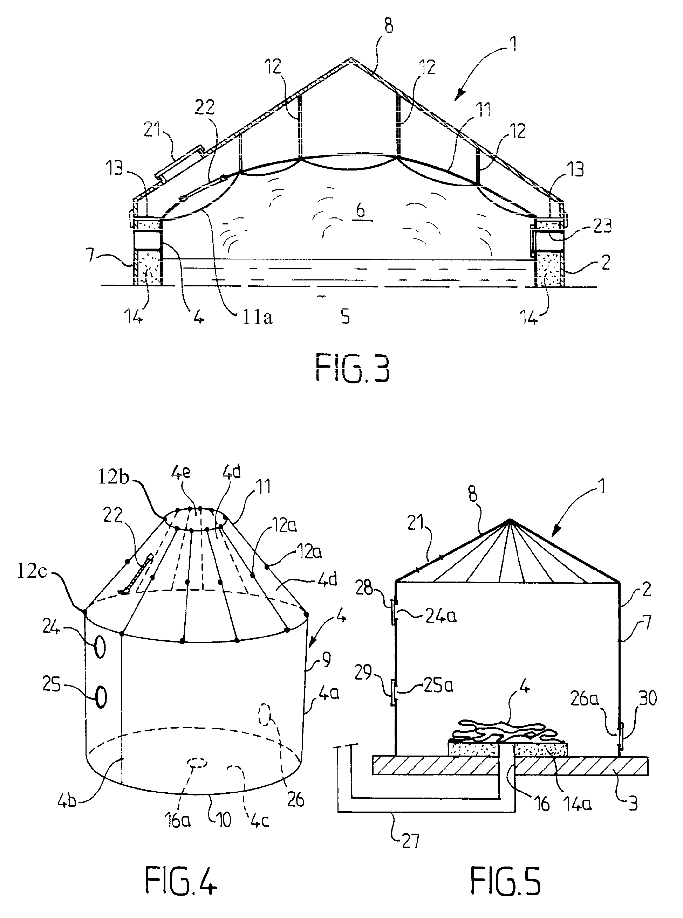

L'enveloppe 4 de confinement des déchets organiques fermentescibles/ciel gazeux, dont un mode de réalisation particulier sera décrit plus en détail ci-après avec référence à la Figure 5, comporte une paroi latérale 9 raccordée à un fond 10 et fermée à sa partie supérieure par une couverture 11.

La partie de l'enveloppe souple 4 enfermant les déchets organiques fermentescibles 5 est représentée sous la forme sensiblement cylindrique qu'elle occupe lorsqu'elle est à l'état rempli, et la partie supérieure de celle-ci, enfermant le ciel gazeux 6, est représentée sous forme libre et souple.

La couverture 11 est reliée au toit 8 du silo 2 par des attaches supérieures verticales 12 et à la région de bordure supérieure de la paroi latérale 7 du silo 2 par des attaches latérales obliques 13. Les attaches 12 et 13 sont réparties régulièrement respectivement sur le dessus et à la périphérie de la couverture 11 et sont constituées par des cordes afin de permettre le libre mouvement de la couverture 11.

Entre la paroi latérale 7 du silo 2 et la paroi latérale 9 de l'enveloppe 4 et entre la surface supérieure de la dalle 3 et le fond 10 de l'enveloppe 4 est disposé un isolant rigide 14, lequel est avantageusement constitué par une mousse de polyuréthane.

Sur la Figure 1, on peut également voir que l'on a représenté des perçages pour l'entrée des déchets organiques fermentescibles dans l'enveloppe 4, la sortie du digestat hors de ladite enveloppe 4, ainsi que la sortie du biogaz.

Sont ici prévus un perçage latéral 15 traversant l'isolant 14 et la paroi latérale 7 du silo 2, un perçage de fond 16 traversant l'isolant 14 et la dalle de béton 3, et un perçage 17 pour la sortie du biogaz 6.

Le perçage 15 est destiné à recevoir un raccord de conduite d'amenée de déchets organiques fermentescibles. Le perçage 16 est destiné à se raccorder à une conduite se trouvant dans le sol pour l'amenée de déchets organiques fermentescibles et/ou la sortie du digestat. On peut ainsi dans un premier temps introduire un lot de déchets organiques fermentescibles par le perçage 16, puis une fois le digestat formé, soutirer celui-ci par ce même perçage 16. Ce dernier sert aussi à l'arrivée d'air de gonflage de l'enveloppe 4 lors de l'installation de celle-ci comme cela sera décrit ci-après.

On pourra aussi utiliser le perçage 15 pour le gonflage de l'enveloppe 4.

Le perçage 17 est destiné à recevoir un raccord de conduite de sortie du biogaz 6.

Si l'on se réfère à la Figure 2, on peut voir que l'on a représenté le digesteur 1 de la Figure 1 comportant, à la périphérie de la paroi 9 de l'enveloppe 4, un serpentin de chauffage constitué par un tuyau 18 destiné à véhiculer de l'eau chaude, enroulé de façon hélicoïdale autour de ladite paroi 9 en étant inséré complètement dans une rainure hélicoïdale 19 formée dans la paroi intérieure de l'isolant 14. Un film 20 conducteur et répartiteur de chaleur, favorisant un rayonnement thermique vers l'intérieur de l'enveloppe 4 recevant les déchets organiques fermentescibles 5, est appliqué contre la paroi intérieure de l'isolant 14 dans sa région de réception du serpentin de chauffage. Ce rayonnement thermique est symbolisé par les flèches f sur la Figure 2a.

Dans un mode de réalisation particulier, le tuyau 18 est en polyéthylène réticulé et le film 20, en aluminium armé polyester.

Sur la Figure 3, on a représenté la partie haute d'un digesteur 1 conforme à un mode de réalisation particulier de l'invention. Le toit 8 de ce digesteur 1 est de forme générale conique, s'effilant vers le haut.

Un trou d'homme 21 est formé dans la partie inclinée du toit 8, au voisinage de sa jonction avec la paroi latérale cylindrique 7 du silo 2.

Une fermeture à glissière 22 est formée dans la couverture 11 de l'enveloppe 4 en regard du trou d'homme 21.

Dans la partie supérieure de la paroi 7, est pratiqué un perçage pour l'installation d'un hublot 23 permettant de surveiller l'état du ciel gazeux 6 et l'état de la surface des déchets fermentescibles 5. En particulier, on peut surveiller qu'il ne se forme pas de croûte à la surface des déchets fermentescibles 5.

Sur la Figure 4, on peut voir l'enveloppe 4 en position déployée. Elle est constituée par :
- une bande rectangulaire 4a qui est soudée en 4b le long de ses deux bords libres de plus petite dimension pour en constituer la paroi latérale 9 ;
- une pièce circulaire 4c constituant le fond 10 soudée à la bordure inférieure de la paroi latérale 9 ; et
- des pièces trapézoïdales 4d destinées à constituer ensemble la couverture 11 en étant fermées à leurs parties supérieures par une pièce circulaire 4e.

Les pièces trapézoïdales 4d sont soudées l'une à l'autre latéralement ; elles sont soudées le long de leurs bordures inférieures à la bordure supérieure de la paroi latérale 9 et le long de leurs bordures supérieures à la pièce circulaire 4e.

La fermeture à glissière 22 est prévue dans l'une des pièces trapézoïdales 4d.

Un trou central 16a est pratiqué dans la pièce 4c pour coopérer avec le perçage 16.

Deux trous 24 et 25 sont également pratiqués l'un au-dessus de l'autre selon une ligne génératrice de la paroi 9 pour constituer, l'un (25), une entrée de matières organiques fermentescibles 5, et l'autre (24), une sortie de biogaz 6.

Un trou 26 est pratiqué dans une région diamétralement opposée de la paroi 9, au voisinage du fond 10, pour constituer également une entrée de matières organiques fermentescibles.

Sur la Figure 4, on a également noté les points 12a de liaison de la couverture 11 avec les attaches 12 reliées au centre de la partie inclinée du toit 8, les points 12b de liaison avec les attaches 12 reliées au bord de la partie supérieure plane 8a du toit 8, et les points 12c de liaison avec les attaches latérales 13.

Sur la Figure 3, on a également représenté un filet 11a attaché par points de façon lâche dans l'enveloppe 4 à la couverture 11. Le filet 11a est destiné à la fixation de bactéries qui se développent dans le ciel gazeux 6 et qui ont la capacité de faire précipiter le soufre présent dans le biogaz.

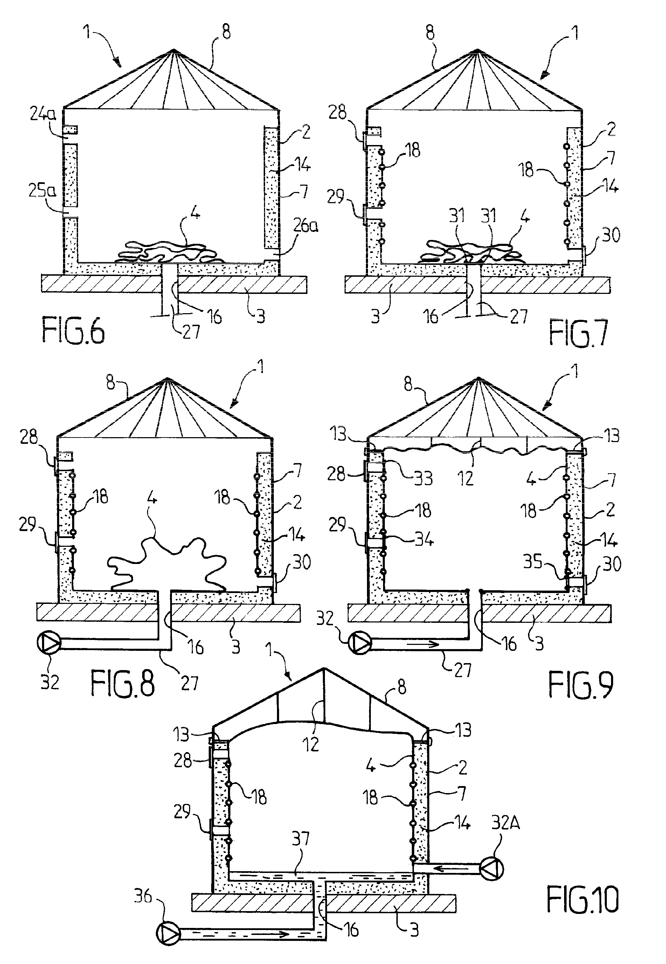

Si l'on se réfère maintenant aux Figures 5 à 10, on peut voir que l'on a illustré les étapes successives de montage du digesteur 1.

### Figure 5

On coule sur le sol une dalle de béton 3 présentant le trou central 16 dans lequel on a fait passer un tuyau souterrain 27 dont l'autre extrémité débouche à la surface du sol à proximité de la dalle de béton 3. Le tuyau 27 est destiné ici à l'évacuation du digestat.

On érige le silo 2 sur la dalle 3.

On laisse ouvert une partie du toit 8 correspondant au trou d'homme 21, ce qui permet à l'installateur d'entrer dans le silo 2 avec le matériel nécessaire pour poursuivre l'installation.

On vient alors poser et fixer sur la dalle 3 une plaque de matière isolante 14a comportant un trou central qui vient en regard du trou 16 et est traversé par l'extrémité saillante du tuyau 27.

On vient poser l'enveloppe 4 dans sa position affaissée sur la plaque 14a en mettant en regard le trou 16a de l'enveloppe avec le trou central de la plaque 14a. Le tuyau 27 fait saillie à l'intérieur de l'enveloppe 4 (ce qui n'a pas été représenté). On fixe la partie du fond de l'enveloppe 4 entourant le trou 16a à la plaque 14a.

On obture les ouvertures 24a, 25a et 26a du silo 2 correspondant aux ouvertures respectivement 24, 25 et 26 de l'enveloppe 4 à l'état complètement déployé à l'aide de plaques amovibles respectivement 28, 29 et 30.

### Figure 6

On vient compléter l'installation de l'isolant 14 par projection de l'isolant liquide sur le reste de la dalle 3 et sur la paroi 7 du silo 2, l'isolant liquide durcissant pour former la couche de mousse isolante s'accrochant sur la dalle 3 et la paroi 7.

Après projection et séchage, on creuse la couche de mousse dans les parties en regard des plaques respectivement 28, 29 et 30 correspondant aux ouvertures 24a, 25a et 26a pour la sortie (24a) de biogaz et l'entrée (25a; 26a) de matières organiques fermentescibles.

### Figure 7

Dans l'isolant 14, on creuse la rainure hélicoïdale 19 pour le passage du tuyau hélicoïdal d'eau chaude 18. On pose la feuille mince d'aluminium 20 sur toute la surface interne verticale de l'isolant 14, cette feuille qui est souple entrant dans la rainure hélicoïdale 19 en tapissant ladite surface interne verticale.

On vient alors incruster dans la paroi latérale interne de l'isolant 14 le serpentin d'eau chaude 18.

On relie l'enveloppe 4 au tuyau 16 par une bride 31, si cela n'a pas déjà été effectué précédemment.

### Figure 8

On commence à gonfler la membrane 4 à l'aide du gonfleur à air 32 disposé sur le tuyau 27.

### Figure 9

On fixe les attaches 13 et on rentre dans l'enveloppe 4 par la fermeture à glissière 32 pour placer les brides de fixation 33, 34 et 35 autour des trous respectivement 24, 25 et 26 de l'enveloppe 4.

On continue le gonflage de l'enveloppe 4 à l'aide du gonfleur à air 32.

### Figure 10

On vient mettre en place les attaches supérieures 12.

On remplace le gonfleur à air 32 par une pompe à eau 36 et on introduit de l'eau au fond de l'enveloppe 4 pour mieux positionner celle-ci et la plaquer contre le fond du silo 2 revêtu de son isolant.

On vient disposer sur la sortie 26a un tuyau d'entrée d'air permettant la poursuite et la fin du gonflage de l'enveloppe 4 par le gonfleur à air 32A.

### Figure 11

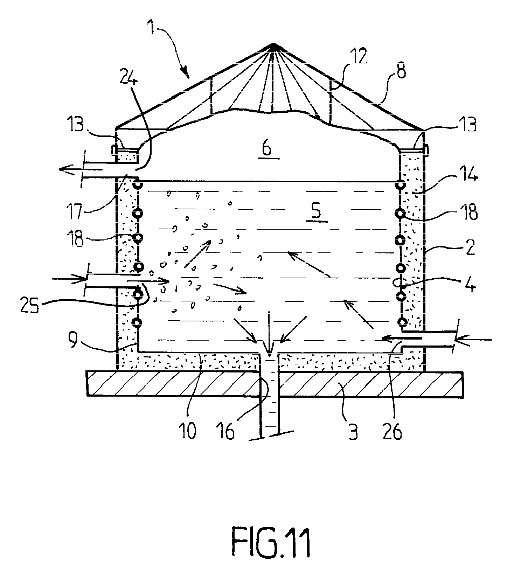

Si l'on réfère maintenant à la Figure 11, on peut voir que l'on a représenté le digesteur 1 en situation de fonctionnement. Dans l'exemple représenté, les matières fermentescibles sont amenées par les trous 25 et 26 dans l'enveloppe 4, le digestat sortant par le perçage 16 du fond de l'enveloppe 4 et le biogaz 6, par le trou 24 de l'enveloppe 4.

## Revendications

1. Digesteur (1) pour la production de biogaz et de digestat, comprenant un silo externe (2) dans lequel est logé un réceptacle pour les matières organiques fermentescibles (5), dont la fermentation conduit d'une part à un digestat restant dans ledit réceptacle et, d'autre part à un biogaz (6) se formant au-dessus desdites matières organiques fermentescibles (5) qui se transforment en digestat, ledit silo (2) comportant au moins une entrée desdites matières organiques fermentescibles (5) et au moins une sortie dudit biogaz (6), **caractérisé par le fait que** le réceptacle pour les matières organiques fermentescibles (5) consiste en une enveloppe souple (4), fermée sur elle-même, de confinement desdites matières organiques fermentescibles (5) et dudit biogaz (6), la ou les entrées de matières organiques fermentescibles (5) et la ou les sorties de biogaz (6) traversant ladite enveloppe (4) de manière étanche.

2. Digesteur selon la revendication 1, **caractérisé par le fait que** l'enveloppe souple (4) est une bâche souple ou une géomembrane consistant en une toile ou un tissu ou géotextile enduit notamment d'un poly(chlorure de vinyle) (PVC).

3. Digesteur selon l'une des revendications 1 et 2, **caractérisé par le fait que** l'enveloppe souple (4) est formée par l'assemblage de pièces par soudage.

4. Digesteur selon la revendication 3, comportant un silo externe (2) de forme générale cylindrique, **caractérisé par le fait que** l'enveloppe souple (4) est formée par soudage d'un fond circulaire (4c), d'une bande rectangulaire (4a) soudée le long de ses deux bordures pour former une paroi latérale, et d'au moins une pièce de couverture (4d-4e).

5. Digesteur selon la revendication 4, **caractérisé par le fait que** la partie de couverture (11) de l'enveloppe souple (4) se situant au-dessus du biogaz (6) porte au moins une sangle (12; 13) de liaison à un toit (8) du silo externe (2).

6. Digesteur selon l'une des revendications 1 à 5, **caractérisé par le fait que** l'enveloppe souple (4) comporte dans sa partie de couverture (11) au moins une ouverture refermable hermétiquement (22) et le silo externe (2) comporte dans un toit (8) de celui-ci au moins un trou d'homme (21) en regard d'une ouverture refermable hermétiquement (22).

7. Digesteur selon l'une des revendications 1 à 6, **caractérisé par le fait que** l'enveloppe souple (4) porte intérieurement, rattaché par points ou par lignes, à sa partie de couverture (11), un filet (11a) pour la fixation de bactéries qui se développent dans le biogaz (6) et qui ont la capacité de faire précipiter le soufre présent dans celui-ci.

8. Digesteur selon l'une des revendications 1 à 7, **caractérisé par le fait qu'**une couche de matière isolante (14) est formée entre le silo externe (2) et l'enveloppe de confinement (4), excepté entre la partie de couverture (11) de l'enveloppe (4) et le toit (8) du silo (2).

9. Digesteur selon la revendication 8, **caractérisé par le fait qu'**un dispositif de chauffage consistant en un serpentin hélicoïdal (18) dans lequel circule de l'eau chaude est incorporé dans ladite couche de matière isolante (14) de façon à entourer la paroi latérale (9) de l'enveloppe (4), une rainure hélicoïdale (19) étant pratiquée dans ladite couche de matière isolante (14) pour recevoir le serpentin (18) et un film mince métallique conducteur (20) étant avantageusement appliqué au fond de ladite rainure (19).

10. Procédé de montage du digesteur tel que défini à l'une des revendications 1 à 9, **caractérisé par le fait que** :
- on coule sur le sol une dalle de béton, on monte un silo sur la dalle de béton et on fait déboucher un tuyau dans le silo à travers la dalle de béton sur laquelle on a posé une plaque isolante, on pose une enveloppe souple non gonflée sur ladite plaque ;
- on raccorde ladite enveloppe et ledit tuyau pour que celui-ci débouche de manière étanche dans ladite enveloppe et on complète l'isolation du fond et de la paroi du silo, on pratique des trous dans l'isolant en regard des ouvertures prévues dans le silo pour l'entrée des matières fermentescibles et la sortie du biogaz ;
- on referme ces ouvertures et on place contre la paroi latérale de l'isolation un serpentin de passage d'eau chaude de préférence avec interposition d'une feuille métallique entre ladite paroi et le serpentin ;
- on gonfle l'enveloppe par un gonfleur à air placé sur le tuyau débouchant dans celle-ci ;
- on raccorde l'enveloppe de manière étanche sur les ouvertures précitées et on continue à gonfler ladite enveloppe ; et
- on attache ladite enveloppe au silo en partie supérieure.
